# FASCICULE DE BREVET EUROPEEN

(11) **EP 2 411 016 B1**
(45) Date de publication et mention de la délivrance du brevet: **14.02.2018**
(21) Numéro de dépôt: 10711440.7
(22) Date de dépôt: 22.03.2010
(51) Int. Cl.: A61K 31/702, A61K 36/48, A23L 29/206, A61P 1/00, A23L 11/00

(54) **EXTRAIT HYDROSOLUBLE DE POIS DÉFRUCTOSYLÉ ET SON UTILISATION COMME AGENT PRÉBIOTIQUE**
WASSERLÖSLICHER DEFRUCTOSYLIERTER ERBSENEXTRAKT UND VERWENDUNG DAVON ALS PRÄBIOTIKUM
WATER SOLUBLE DEFRUCTOSYLATED PEA EXTRACT, AND USE THEREOF AS A PREBIOTIC AGENT

(30) Priorité: 27.03.2009 FR 0901511
(43) Date de publication de la demande: 01.02.2012
(73) Titulaire: Olygose, 60280 Venette (FR)
(72) Inventeur: Delbaere, Francois, 60200 Compiegne (FR)
(74) Mandataire: Demulsant, Xavier
(86) Numéro de dépôt international: PCT/FR2010/000238
(87) Numéro de publication internationale: WO 2010/109093

(56) Documents cités:
- WO-A-2007/017572
- FR-A- 2 897 239
- US-A- 4 216 235

## Description

La présente invention concerne un extrait hydrosoluble de pois défructosylé, sensiblement exempt de protéines et de peptides, ainsi qu'une composition d'oligosaccharides telle que celle contenue dans ledit extrait. Elle concerne également leur procédé de préparation, ainsi que leurs utilisations en tant qu'agent prébiotique.

Les légumineuses constituent une matière première de choix pour l'industrie agro-alimentaire, notamment pour la production de protéines, d'amidon notamment riche en amylose, de fibres et de dérivés de l'amidon tels que les sirops de glucose, la maltodextrine, le dextrose ou l'isoglucose.

Ces produits trouvent des débouchés dans des domaines variés, tels que les secteurs des adhésifs ou du papier, mais surtout dans le domaine alimentaire, où l'intérêt nutritionnel des légumineuses, aussi bien dans l'alimentation humaine qu'animale, n'est plus à démontrer. Parmi celles-ci, les légumineuses à graines, telles que le haricot, le pois et la fève, sont ainsi largement utilisées pour leur apport en énergie et en protéines. Les graines de pois sec sont en effet riches en glucides, formés essentiellement d'amidon et également de saccharose et d'oligosaccharides, en protéines (à teneur élevée en lysine) et en fibres.

En contrepartie de leurs bénéfices nutritionnels, les légumineuses tels que le pois sec présentent une mauvaise digestibilité qui nécessite souvent de les faire tremper en milieu acide avant de les cuire et de les consommer. Cet inconvénient est principalement attribué à leur teneur significative en α-galactosyl oligosaccharides constitués d'unités D-galactose, D-glucose et D-fructose. En effet, ces oligosaccharides, qui ne sont pas digestibles par les enzymes humaines (incapables de dégrader leurs liaisons α-1,6-galactosidiques et β-1-4 fructosidiques), sont transportés intacts jusque dans le côlon où ils fournissent un substrat pour la fermentation de bactéries telles que *Clostridium perfringens,* provoquant des phénomènes de flatulence. Ce phénomène a notamment été attribué, selon certains auteurs, dans le cas des haricots (*Phaseolus vulgaris*), au motif terminal fructose du raffinose qu'ils contiennent (MYHARA RM et al., Can. Inst. Food Sci. Technol. J., Vol. 21, n° 3, pp. 245-250, 1988).

Ces oligosaccharides sont donc généralement éliminés soit par voie de sélection agronomiques de lignées (notamment de soja ou de haricot) à teneur réduite en tels oligosaccharides (BURBANO C. et al., J. Sci. Food Agric., Vol. 79, pp. 1468-1472, 1999), soit par séparation et élimination physique, soit encore par hydrolyse enzymatique (à l'aide d'une α-galactosidase) ou fermentaire, effectuée en général préalablement à la consommation de ces légumineuses, mais aussi par administration de compléments alimentaires constitués d'enzymes destinées à hydrolyser ces oligosaccharides en des composés digestibles, avant leur arrivée dans le côlon (US-5,651,967).

Le document US-4,008,334 propose ainsi un procédé pour éliminer les carbohydrates solubles des protéines végétales, issues en particulier du soja, dont le raffinose et le stachyose, par digestion enzymatique à l'aide d'une levure boulangère. De manière analogue, le document US-4,216,235 suggère l'utilisation d'une levure *Saccharomyces uvarum* pour dégrader les oligosaccharides du soja, y compris le mélibiose et le manninotriose.

Dans le cas du pois, les oligosaccharides sont le plus souvent éliminés lors de la transformation des graines de légumineuses par les industriels. On estime qu'à l'heure actuelle, le volume d'oligosaccharides générés par le raffinage du pois est en forte croissance et supérieur à 25.000 t par an en Europe et au Canada. Afin d'éviter d'avoir à les éliminer en station d'épuration, et malgré leur faible digestibilité, ceux-ci sont en général cédés aux agriculteurs pour l'alimentation du bétail. Cette solution est néanmoins coûteuse en énergie car du fait de la très grande dilution de l'extrait soluble issu du raffinage, il est nécessaire de le concentrer pour en assurer la conservation et permettre son transport et son utilisation par les animaux.

Il serait donc intéressant de pouvoir disposer d'un moyen permettant de valoriser ces sous-produits.

Dans cette optique, il a été suggéré dans le document US 2004/0198965 d'utiliser les oligosaccharides, présents notamment dans les graines de soja, pour la synthèse de D-galactose.

L'invention vise à proposer une autre utilisation de ces sous-produits. Précisément, il est apparu au Demandeur qu'une fraction soluble de pois, sensiblement exempte de protéines et peptides et riche en oligosaccharides, pouvait, après défructosylation des oligosaccharides, être utilement exploitée en tant qu'agent prébiotique.

Par « prébiotique », on entend un composant alimentaire, non digestible, fermenté sélectivement, qui induit des changements spécifiques de la composition et/ou de l'activité de la microflore intestinale, conférant des bénéfices sur la santé et le bien-être de l'hôte (Gibson GR et al., Nutrition Research Reviews, 17 : 259-275, 2004). Le prébiotique peut notamment être considéré comme un aliment pour les bactéries favorables du côlon, telles que les bifidobactéries et les lactobacilles, qui permettent de prévenir les troubles intestinaux, d'améliorer l'absorption des minéraux, de moduler le métabolisme lipidique, et/ou de stimuler le système immunitaire.

Depuis qu'on a découvert qu'il était possible de moduler l'équilibre de la flore intestinale par ingestion d'ingrédients alimentaires, de nombreux candidats à l'appellation « prébiotique » ont été étudiés. La plupart sont des glucides d'origine végétale. Les plus connus et les mieux caractérisés sont les fructanes, polymères de fructose, parmi lesquels on trouve l'inuline, généralement extraite de tubercules de chicorée, mais encore d'agave, et les fructo-oligosaccharides (FOS) produits soit par hydrolyse de l'inuline, soit par biosynthèse à partir de saccharose et de fructose.

D'autres oligosaccharides possèdent aussi des propriétés prébiotiques plus ou moins établies: les galacto-oligosaccharides (GOS) ou « trans-galacto-oligosaccharides » (TOS), très proches des composés présents dans le lait maternel, les oligosaccharides de soja (SOS), les isomalto-oligosaccharides (IMOS), le lactulose, le raffinose, les xylo-oligosaccharides, etc.

Malgré les très nombreux travaux réalisés tant sur les prébiotiques bien caractérisés (tels que les FOS et GOS) que sur un grand nombre de molécules candidates, il manque à ce jour un prébiotique combinant les propriétés suivantes qui sont recherchées par les producteurs (potentiels) d'aliments et boissons à fonctionnalité santé : procédé de production simple et économique, très bonne tolérance intestinale permettant l'emploi de concentrations suffisantes pour obtenir en pratique les effets escomptés, et stabilité élevée aux procédés de chauffage et aux milieux acides typiques de larges secteurs de l'industrie alimentaire.

Ainsi, les inulines et FOS présentent une tolérance intestinale moyenne. Selon certains auteurs, les symptômes d'inconfort avec les FOS apparaissent dès 2.5 g/jour pour 25% des sujets, et concernent 75% des sujets à 20g/j. La tolérance des inulines est comparable à celle des FOS, mais leur dose efficace pour obtenir un effet bifidogène est plus élevée, ce qui accentue le problème d'arbitrage entre dose minimale pour obtenir un effet bifidogène et dose maximale pour éviter les problèmes de tolérance intestinale. En outre, les inulines et les FOS ne sont pas stables aux acides et à la chaleur.

Il n'en est pas de même des XOS, du raffinose et des SOS qui offrent en outre une bonne activité prébiotique. Ceux-ci présentent toutefois une tolérance intestinale très faible.

Les pyrodextrines, IMOS et polydextrose, qui présentent une bonne stabilité aux acides et à la chaleur et une bonne tolérance, présentent en revanche une efficacité faible du fait d'une faible spécificité prébiotique (leur stimulation des bactéries coliques est indifférenciée) et/ou d'une faible indigestibilité.

De leur côté, les GOS présentent une tolérance intestinale et une stabilité à l'acidité et aux procédés élevée. Ils sont obtenus par réaction de trans-galactosylation (polymérisation) à partir de lactose au moyen de β-galactosidases. Le rendement de réaction est faible, négativement impacté par la synthèse des composés recherchés (β-GOS), de sorte que diverses technologies de séparation, notamment membranaires ou chromatographiques, sont nécessaires sur le produit de réaction pour permettre de maintenir des conditions favorables à la trans-galactosylation et à l'obtention d'un produit de pureté suffisante à un usage prébiotique. Ces technologies sont coûteuses et vont donc à l'encontre d'une offre abordable de GOS prébiotiques.

Il en résulte que les prébiotiques existants ne sont utilisés que dans un nombre limité d'applications alimentaires (laits maternisés en particulier) et à des concentrations qui ne permettent pas toujours de garantir les effets théoriquement possibles selon la recherche in vitro/vivo voire clinique.

Or, le Demandeur a mis en évidence que les mélanges d'oligosaccharides présents dans les extraits de pois défructosylés permettaient d'obtenir le compromis de propriétés recherché pour les prébiotiques.

La présente invention a ainsi pour objet un extrait hydrosoluble de pois défructosylé, renfermant moins de 5 % en poids, sur matière sèche, de protéines et de peptides. Elle a également pour objet une composition ayant le profil d'oligosaccharides de ces extraits hydrosolubles de pois défructosylé et qui renferme, et de préférence est constituée essentiellement, du mélibiose, du manninotriose et du manninotétraose dans un rapport en poids manninotétraose/mélibiose d'au moins 1:1 et de préférence d'au moins 4:1 voire d'au moins 5:1 (et au maximum d'environ 10:1, par exemple) et/ou un rapport en poids manninotriose/manninotétraose de 0,3:1 à 4:1, de préférence de 0,8:1 à 1:1.

Elle a encore pour objet l'utilisation non-thérapeutique comme agent prébiotique soit d'un extrait hydrosoluble de pois défructosylé, renfermant moins de 5 % en poids, sur matière sèche, de protéines et de peptides, soit de la composition précitée.

Par "essentiellement exempt de protéines et peptides", on entend que l'extrait hydrosoluble selon l'invention renferme moins de 5% en poids (sur matière sèche) de tels constituants. Ceux-ci incluent notamment les albumines.

L'extrait de pois défructosylé selon l'invention peut être obtenu par défructosylation des oligosaccharides présents dans les solubles de pois sensiblement exempts de protéines et de peptides.

Le procédé d'obtention de cet extrait peut notamment comprendre les étapes suivantes :
1- Séparation des protéines floculables, fibres insolubles et/ou amidon du pois ou d'une farine de pois, pour obtenir un extrait hydrosoluble de pois,
2- Elimination des peptides solubles, en particulier des facteurs antitrypsiques,
3- Action d'une invertase, issue par exemple de *Saccharomyces cerevisiae,* sur ledit extrait, dans des conditions permettant la défructosylation des oligosaccharides présents dans ledit extrait, et
4- Récupération de l'extrait défructosylé ainsi obtenu.

L'invention a donc également pour objet un procédé de préparation d'un extrait hydrosoluble de pois défructosylé sensiblement exempt de protéines et de peptides, comprenant au moins les étapes successives décrites ci-dessus.

Il est bien entendu que ce procédé peut, comme expliqué ci-dessous, comprendre d'autres étapes préliminaires, intermédiaires ou subséquentes à celles indiquées ci-dessus. Il peut ainsi notamment inclure au moins une étape de déminéralisation (désalage) pour diminuer les réactions de coloration dans certaines applications. Avant élimination des autres minéraux, on applique avantageusement une étape d'élimination de la potasse (par cristallisation par exemple), similaire à celle opérée sur les vinasses de sucrerie. Les sels présents dans les solubles de pois étant particulièrement riches en potassium (de l'ordre de 35%), ce traitement présente le double avantage de diminuer de 50% environ la teneur en minéraux restant à éliminer par d'autres techniques (notamment résines échangeuses d'ions) et de générer de la potasse qui constitue un engrais de valeur élevée, autorisé en culture biologique. Ce traitement consiste généralement en une acidification (souvent par H2SO4) suivie d'une neutralisation à l'ammoniaque, les cristaux étant récupérés par décantation et/ou centrifugation. En variante, on peut utiliser les techniques membranaires (notamment l'électrodialyse) qui permettent d'optimiser le procédé en diminuant les coûts.

Un tel procédé est généralement mis en oeuvre sur du pois natif. Le pois utilisé selon l'invention peut être une variété de pois lisse ou ridé et il est avantageusement choisi parmi ceux de l'espèce *Pisum sativum* L., notamment parmi les sous-espèces : *elatius, transcaucasicum* Govorov, *syriacum* Berger, *abyssinicum* Govorov, *asiaticum* Govorov, *sativum* (variétés *Arvense* L., sativum ou *macrocarpon,* par exemple).

Les pois peuvent être préalablement nettoyés, triés, émondés et/ou dépoussiérés avant d'être broyés en une farine qui est ensuite mise en suspension dans l'eau.

La séparation des protéines floculables, de l'amidon et/ou des fibres insolubles peut se faire par tout moyen connu de l'homme du métier et notamment par extraction, centrifugation, décantation, filtration sur membrane (notamment ultrafiltration) ou précipitation isoélectrique des protéines ou par une combinaison de plusieurs de ces moyens, éventuellement combinés à une étape de concentration, par exemple par osmose inverse et/ou évaporation sous vide. Un exemple d'un tel procédé de séparation est décrit dans la demande WO 2007/017572.

Les solubles de pois obtenus sont riches en peptides et en oligosaccharides. Ces solubles de pois sont traités pour séparer les peptides, qui présentent un poids moléculaire supérieur à 5000 Da, avantageusement par ultrafiltration et/ou nanofiltration. Il est à noter que la fraction peptidique récupérable est enrichie en albumine PA1b (de poids moléculaire 11.000 Da) dont les propriétés insecticides appropriées aux céréales sont décrites dans la demande EP 1 078 085. La récupération de cette fraction pour un tel usage non alimentaire constitue donc un avantage potentiel supplémentaire de la présente invention.

A l'issue de cette étape, on obtient une fraction hydrosoluble riche en oligosaccharides et sensiblement exempte de protéines et de peptides, qui peut avantageusement être concentrée par osmose inverse.

La défructosylation des oligosaccharides de pois résiduels contenus dans cette fraction peut ensuite être réalisée, de façon continue ou séquentielle, par tout procédé d'hydrolyse acide, thermique et/ou enzymatique permettant d'éliminer le motif fructose terminal des oligosaccharides présents dans les solubles de pois, tout en préservant leurs liaisons α-galactosidiques, et en particulier en les soumettant à l'action d'une enzyme ou d'une levure (avantageusement *Saccharomyces cerevisiae*) présentant une activité invertase (ou β-fructofuranosidase). Cette étape permet de transformer respectivement le raffinose, le stachyose et le verbascose en mélibiose, manninotriose et manninotétraose.

L'enzyme libre (invertase de *Saccharomyces cerevisiae*) peut par exemple être mise en oeuvre en quantité de 200 à 300 UI.mg⁻¹ sur sec, à une température de 40 à 50°C, avantageusement à un pH de 5,0 à 5,4, pendant une durée allant par exemple de 10 à 14h, sur un perméat concentré à 100-400 g/l, par exemple d'environ 200 g/l, à raison de 50 à 70 UI d'enzyme par gramme de matière sèche du perméat.

L'utilisation d'une levure, plutôt que d'une enzyme, dans ce procédé de défructosylation présente l'avantage que la levure utilise, et de ce fait élimine, le fructose produit par la réaction, ce qui permet de récupérer directement (sans séparation ultérieure) un produit sans sucre. Dans le cas où le procédé selon l'invention utilise une enzyme, il comprendra au contraire une étape supplémentaire d'élimination du fructose.

Ce procédé présente des avantages environnementaux non négligeables par rapport aux procédés d'obtention de prébiotiques de l'art antérieur, dans la mesure où :
- Les matières premières utilisées sont de préférence des déchets de l'industrie agro-alimentaire,
- Il fait avantageusement appel à des techniques de purification par membrane, qui sont peu consommatrices d'énergie,
- Il permet d'avoir recours à des biotechnologies naturelles, en utilisant des micro-organismes traditionnels non modifiés.

L'extrait soluble de pois défructosylé obtenu peut être utilisé sous forme liquide ou éventuellement sous forme pulvérulente après avoir été déshydraté, notamment par atomisation ou lyophilisation. Son emploi sous forme liquide est préféré, puisque grâce à son faible poids moléculaire moyen et à sa polydispersité, le produit offre une faible viscosité, n'engendre pas de problèmes de cristallisation et offre une bonne stabilité microbiologique.

Il a été démontré que le profil d'oligosaccharides du pois défructosylé était particulièrement avantageux sur le plan de l'efficacité prébiotique recherchée et de sa tolérance, par rapport à des mélanges d'oligosaccharides issus d'autres légumineuses défructosylées, telles que le soja ou le haricot. Notamment, l'effet laxatif du pois est inférieur à celui du soja et il présente des effets prébiotiques non seulement à l'entrée du côlon, mais aussi dans le côlon proximal et médian. Le procédé de défructosylation est en outre moins complexe et coûteux dans le cas du pois que des autres légumineuses telles que le soja et le haricot, dans la mesure où il ne nécessite pas d'étape de séparation du sucrose, ni de déminéralisation qui est au contraire indispensable dans le cas du soja, dont les solubles sont très riches en sels minéraux.

En outre, en raison de l'élimination du fructose, l'extrait de pois défructosylé selon l'invention est hypocariogène et présente une faible valeur calorique.

L'extrait de pois défructosylé selon l'invention, et les compositions analogues, constituent donc un ingrédient prébiotique de choix, en vue d'obtenir au moins l'un des bénéfices suivants : modification de la composition bactérienne du côlon, stimulation des bifidobactéries et lactobacilles, diminution de la quantité relative des bactéries néfastes et germes pathogènes tels que *Clostridium, E.coli,* diminution du pH luminal, augmentation de l'absorption minérale, en particulier du calcium, stimulation des enzymes bactériennes détoxifiantes, répression des enzymes bactériennes toxifiantes, insolubilisation des sels biliaires favorisant leur excrétion, inhibition de la conversion des acides biliaires, diminution de la production de composés néfastes tels que phénols et indoles, diminution de la constipation, réduction du risque de diarrhées infectieuses, réduction des maladies inflammatoires intestinales, stimulation de la production d'acides gras à chaînes courtes, stimulation de l'apoptose, prévention du cancer du côlon, renforcement de la barrière intestinale, diminution de la translocation bactérienne et/ou de toxines bactériennes, stimulation de la sécrétion d'hormones de satiété, diminution du taux de cholestérol, réduction du poids, diminution de la prise de poids, diminution de la prise alimentaire, réduction de l'adipogénèse et/ou réduction des symptômes de dérèglement métabolique.

Ils sont donc avantageusement utilisés à ces fins, ou pour la fabrication d'une composition destinée à obtenir l'un au moins des bénéfices précités.

Par ailleurs, en raison de sa simplicité d'obtention et de son faible coût de fabrication, l'extrait de pois défructosylé selon l'invention convient bien à une utilisation dans des produits de grande consommation. Il est particulièrement bien adapté à une utilisation dans des aliments longue conservation et des boissons carbonatées à saveur sucrée, compte tenu de sa stabilité au stockage, à la chaleur et aux acides.

Dans ces applications, la défructosylation de l'extrait de pois peut être effectuée préalablement à sa mise en oeuvre dans le produit alimentaire ou la boisson auxquels il est destiné à conférer des propriétés prébiotiques. En variante, la défructosylation peut être réalisée durant le traitement de préparation et/ou au cours du stockage (sous l'action de l'acidité ou de l'activité invertase présente dans ce produit alimentaire ou dans cette boisson).

La présente invention a donc également pour objet une boisson carbonatée renfermant au moins un édulcorant et un extrait hydrosoluble de pois défructosylé et/ou une composition telle que décrite précédemment.

Elle a également pour objet un aliment, tel qu'un aliment longue conservation, un yoghourt ou un lait infantile, renfermant un extrait hydrosoluble de pois défructosylé et/ou une composition telle que décrite précédemment.

En variante, l'extrait de pois défructosylé selon l'invention, ou la composition correspondante, peut être administré en tant que complément nutritionnel, par exemple sous forme de sirop, de gélule, de comprimé, de poudre pour suspension buvable ou de toute autre forme galénique convenant à une administration par voie orale.

Selon un mode de réalisation de l'invention, la boisson, l'aliment ou le complément nutritionnel renfermant l'extrait de pois défructosylé selon l'invention, ou la composition correspondante, peut en outre inclure au moins un autre agent prébiotique, notamment issu de solubles de soja défructosylés, de féverole défructosylée ou de vinasses de betteraves défructosylées.

L'invention sera maintenant illustrée par les exemples non limitatifs suivants.

### EXEMPLES

### Exemple 1 : Préparation d'un extrait hydrosoluble de pois défructosylé

Une fraction hydrosoluble de pois à 50% en poids de matière sèche, obtenue après extraction de l'amidon et des fibres et coagulation des protéines, est diluée à 15% en poids de matière sèche et filtrée au moyen d'une membrane d'ultrafiltration, avec un seuil de coupure fixé à 5.000 Da, en vue de la clarifier et d'en éliminer les peptides. Cette étape est suivie d'une concentration du perméat par osmose inverse, pour le ramener à 20% en poids de matière sèche.

Parallèlement, on prépare 100 ml d'une solution d'invertase à 1 mg/ml, qui est ensuite également lavée par centrifugation pendant 30 minutes. Le culot est repris par 50 ml d'eau. On mélange alors 980 ml de la fraction de pois avec 50 ml de la solution enzymatique, dans un réacteur à double paroi et agitateur placé au bain-marie à 50°C. L'hydrolyse est contrôlée par dosage des sucres réducteurs à l'aide d'une solution alcaline aqueuse d'acide 3,5-dinitrosalicylique (DNS), à différents intervalles de temps. Après au moins 12h d'hydrolyse, l'enzyme est neutralisée, puis le produit obtenu est centrifugé puis filtré pour obtenir une solution limpide qui est ensuite concentrée par évaporation rotative sous vide à 70°C, jusqu'à l'obtention d'un jus limpide.

### Exemple 2: Etude de tolérance digestive de l'extrait hydrosoluble de pois défructolysé

### 2-1 : Objectif de l'étude

L'objectif de cette étude a été de comparer la tolérance digestive du produit issu de la présente invention (oligosaccharides défructolysés : OD) à celle de deux prébiotiques déjà présents sur le marché et à celle d'un placebo.

Cette étude de tolérance digestive a été réalisée en France sur 17 personnes volontaires (8 hommes et 9 femmes) en double aveugle.

### 2-2 : Protocole opératoire

Les volontaires ayant participé à cette étude ont consommé, de façon randomisée, 20g de substance active diluée dans du jus d'orange en une prise, chaque prise étant espacée par un temps de repos de 48 heures, pour atteindre quatre prises au total.

Les différents produits testés étaient :
- des oligosaccharides défructolysés (OD : produit de l'invention),
- des β-galacto-oligosaccharides (β-GOS),
- des fructo-oligosaccharides (FOS),
- du saccharose (placebo)

La tolérance digestive a été évaluée grâce à un questionnaire renseignant la fréquence (notée de 1 : pas de symptôme à 5 : symptômes très fréquents) et l'intensité (notée de 1 : pas de symptôme ressenti à 5 : symptômes importants et/ou inacceptables) de 6 paramètres différents :
- douleurs abdominales,
- ballonnements,
- bruits abdominaux (borborygmes, gargouillis...),
- flatulence,
- nausées, régurgitation ou brûlures d'estomac,
- besoin de déféquer

### 2-3 : Résultats et conclusions de l'étude

Les résultats de l'étude sont présentés dans le tableau 1 ci-après, qui regroupe la moyenne des notes obtenues pour chaque paramètre suite à l'évaluation de la tolérance digestive des OD, des β-GOS et des FOS par rapport à celle d'un placebo.

**Tableau 1**

| | Douleurs abdominales | | Ballonnement s | | Bruits abdominaux | | Flatulence | | Nausées | | Besoin de déféquer | | Note cumulée |
|---|---|---|---|---|---|---|---|---|---|---|---|---|---|
| | F | I | F | I | F | I | F | I | F | I | F | I | |
| placebo | 1,1 ^{a} | 1,1 ^{a} | 1,2 ^{a} | 1,2 ^{a} | 1,2 ^{a} | 1,2 ^{a} | 1,9 ^{a} | 1,5 ^{a} | 1,0 ^{a} | 1,0 ^{a} | 1,6 ^{a} | 1,2 ^{a} | 15,1 ^{a} |
| OD | 1,4 ^{a} | 1,4 ^{a} | 1,9 ^{b} | 1,5 ^{ab} | 2,0 ^{b} | 1,8 ^{b} | 3,1 ^{b} | 2,6 ^{b} | 1,2 ^{a} | 1,2 ^{a} | 2,0 ^{a} | 1,8 ^{a} | 21,9 ^{b} |
| β-GOS | 1,6 ^{ab} | 1,5 ^{ab} | 1,6 ^{ab} | 1,7 ^{b} | 1,8 ^{b} | 1,6 ^{ab} | 2,9 ^{b} | 2,5 ^{b} | 1,2 ^{a} | 1,1 ^{a} | 2,1 ^{a} | 1,7 ^{a} | 21,3 ^{b} |
| FOS | 2,0 ^{b} | 1,9 ^{b} | 2,1 ^{b} | 1,9 ^{b} | 2,1 ^{b} | 2,0 ^{b} | 3,2 ^{b} | 2,7 ^{b} | 1,0 ^{a} | 1,0 ^{a} | 2,1^{a} | 2,1 ^{a} | 24,2 ^{b} |

| | | | | | | | | | | | | | |
|---|---|---|---|---|---|---|---|---|---|---|---|---|---|
| F : fréquence des symptômes ; I : intensité des symptômes. ^{a, b} : les valeurs des produits portant une lettre différente sont significativement différentes des autres valeurs (p<0.05 ; test de différenciation de Duncan) | | | | | | | | | | | | | |

Comme il ressort du Tableau 1, la fréquence et la sévérité des symptômes observés lors de la consommation des différents produits testés ont montré que ces derniers n'induisaient pas d'intolérance digestive (notes cumulées environ égales au tiers de la note maximale).

Toutefois, certaines différences entre les produits ont pu être relevées :
- les OD ont été aussi bien tolérés que les β-GOS (produit présentant la meilleure tolérance parmi les prébiotiques actuellement sur le marché) par rapport au placebo (qui présente les notes les plus faibles pour l'ensemble des symptômes)
- les OD ont montré une meilleure tolérance digestive que les FOS (qui restent le produit le moins bien toléré avec les notes les plus élevées pour l'ensemble des symptômes).

Cette étude met ainsi en évidence la tolérance digestive satisfaisante du produit issu de l'invention.

### Exemple 3: Etude de l'effet bifidogène de l'extrait hydrosoluble de pois défructolysé

Pour évaluer l'effet bifidogène de l'extrait selon l'invention sur la microflore intestinale, on réalise une étude nutritionnelle monocentrique, randomisée, contre placebo, et en double aveugle sur 36 volontaires sains (hommes et femmes de 20 à 45 ans) répartis en deux groupes égaux, en ambulatoire. Le produit selon l'invention est consommé à la dose de 7 g d'oligosaccharides (représentant 95% de la matière sèche) par jour en une prise, tandis que le placebo est constitué d'un sirop de glucose déshydraté consommé à la même dose. La durée du traitement est de 3 semaines. Les bifidobactéries sont dosées en temps réel par PCR quantitative.

### Exemple 4: Etude de stabilité en milieu acide de l'extrait hydrosoluble de pois défructolysé

La stabilité en milieu acide de l'extrait de pois défructosylé selon l'invention est évaluée sur des boissons type cola sans sucre dont le pH est de 2,8. Les essais sont réalisés en comparaison avec un extrait de pois identique mais non défructosylé.

Pour ce faire, la moitié des bouteilles de chacun de ces lots, contenant 2,48 g de l'extrait testé, est stockée à température ambiante et l'autre moitié est stockée à 37°C, pendant un mois.

Des échantillons de chaque lot conservé à chaque température sont prélevés deux fois par semaine. Les échantillons sont analysés par HPLC afin de déterminer la variation de leur concentration en oligosaccharides au cours du temps et donc la stabilité de ces derniers.

## Revendications

1. Extrait hydrosoluble de pois défructosylé, renfermant moins de 5 % en poids, sur matière sèche, de protéines et de peptides.

2. Extrait selon la revendication 1, **caractérisé en ce qu'**il est obtenu à partir de pois choisi parmi ceux de l'espèce Pisum sativum L., notamment parmi les sous-espèces : elatius, transcaucasicum Govorov, syriacum Berger, abyssinicum Govorov, asiaticum Govorov ou sativum.

3. Procédé de préparation d'un extrait hydrosoluble de pois défructosylé selon l'une des revendications 1 et2, comprenant au moins les étapes successives de :
- Séparation des protéines floculables, fibres insolubles et/ou amidon d'une farine de pois, pour obtenir un extrait hydrosoluble de pois,
- Elimination des peptides solubles, en particulier des facteurs antitrypsiques,
- Action d'une invertase, issue par exemple de Saccharomyces cerevisiae, sur ledit extrait, dans des conditions permettant la défructosylation des oligosaccharides présents dans ledit extrait, et
- Récupération de l'extrait défructosylé ainsi obtenu.

4. Composition renfermant, et de préférence constituée, du mélibiose, du manninotriose et du manninotétraose dans un rapport en poids manninotétraose/mélibiose d'au moins 1:1 et de préférence d'au moins 4:1 voire d'au moins 5:1 et/ou un rapport en poids manninotriose/manninotétraose de 0,3:1 à 4:1, de préférence de 0,8:1 à 1:1.

5. Utilisation non-thérapeutique comme agent prébiotique d'un extrait hydrosoluble de pois défructosylé selon l'une des revendications 1 et 2 ou d'une composition selon la revendication 4.

6. Un extrait hydrosoluble de pois défructosylé tel que défini à l'une des revendications 1 et 2 ou une composition telle que définie à la revendication 4 pour son utilisation comme agent prébiotique.

7. Boisson carbonatée renfermant au moins un édulcorant et un extrait hydrosoluble de pois défructosylé selon l'une des revendications 1 et 2 et/ou une composition selon la revendication 4.

8. Aliment, tel qu'un aliment longue conservation, un yoghourt ou un lait infantile, renfermant un extrait hydrosoluble de pois défructosylé selon l'une des revendications 1 et 2 et/ou une composition selon la revendication 4.

## Patentansprüche

1. Defructosylierter wasserlöslicher Erbsenextrakt, enthaltend weniger als 5 Gew.-%, bezogen auf den Feststoffgehalt, Proteine und Peptide.

2. Extrakt nach Anspruch 1, **dadurch gekennzeichnet, dass** es aus Erbsen, die aus denjenigen der Arten Pisum sativum L., insbesondere aus den Unterarten elatius, transcaucasicum Govorov, syriacum Berger, abyssinicum Govorov, asiaticum Govorov oder sativum, ausgewählt sind, erhalten wird.

3. Verfahren zur Herstellung eines defructosylierten wasserlöslichen Erbsenextrakts nach einem der Ansprüche 1 und 2, das mindestens die folgenden aufeinanderfolgenden Schritte umfasst:
- Abtrennung von ausflockbaren Proteinen, unlöslichen Fasern und/oder Stärke aus einem Erbsenmehl zum Erhalt eines wasserlöslichen Erbsenextrakts,
- Entfernung von löslichen Peptiden, insbesondere Antitrypsin-Faktoren,
- Einwirkung einer Invertase, beispielsweise aus Saccharomyces cerevisiae, auf den Extrakt unter Bedingungen, die die Defructosylierung von in dem Extrakt vorliegenden Oligosacchariden erlauben, und
- Gewinnung des so erhaltenen defructosylierten Extrakts.

4. Zusammensetzung, enthaltend und vorzugsweise bestehend aus Melibiose, Manninotriose und Manninotetraose in einem Manninotetraose/Melibiose-Gewichtsverhältnis von mindestens 1:1 und vorzugsweise mindestens 4:1 oder sogar mindestens 5:1 und/oder einem Manninotriose/Manninotetraose-Gewichtsverhältnis von 0,3:1 bis 4:1, vorzugsweise von 0,8:1 bis 1:1.

5. Nichttherapeutische Verwendung eines defructosylierten wasserlöslichen Erbsenextrakts nach einem der Ansprüche 1 und 2 oder einer Zusammensetzung nach Anspruch 4 als präbiotisches Mittel.

6. Defructosylierter wasserlöslicher Erbsenextrakt gemäß einem der Ansprüche 1 und 2 oder Zusammensetzung gemäß Anspruch 4 zur Verwendung als präbiotisches Mittel.

7. Kohlensäurehaltiges Getränk, enthaltend mindestens ein Süßungsmittel und einen defructosylierten wasserlöslichen Erbsenextrakt nach einem der Ansprüche 1 und 2 und/oder eine Zusammensetzung nach Anspruch 4.

8. Lebensmittel, wie ein lange haltbares Lebensmittel, ein Joghurt oder eine Säuglingsmilch, enthaltend einen defructosylierten wasserlöslichen Erbsenextrakt nach einem der Ansprüche 1 und 2 und/oder eine Zusammensetzung nach Anspruch 4.

## Claims

1. Water-soluble defructosylated pea extract containing less than 5% by weight, relative to solids, of proteins and of peptides.

2. Extract according to Claim 1, **characterized in that** it is obtained from peas chosen from those of the species *Pisum sativum* L., especially from the subspecies *elatius, transcaucasicum* Govorov, *syriacum* Berger, *abyssinicum* Govorov, *asiaticum* Govorov or *sativum.*

3. Process for preparing a water-soluble defructosylated pea extract according to either of Claims 1 and 2, comprising at least the successive steps of:
- separation of proteins able to be flocculated, insoluble fibres and/or starch from a pea flour, to obtain a water-soluble pea extract,
- removal of the soluble peptides, in particular antitrypsin factors,
- action of an invertase, resulting for example from *Saccharomyces cerevisiae,* on said extract under conditions enabling the defructosylation of the oligosaccharides present in said extract, and
- recovery of the defructosylated extract obtained in this way.

4. Composition containing, and preferably consisting of, melibiose, manninotriose, and manninotetraose, in a manninotetraose/melibiose weight ratio of at least 1:1 and preferably of at least 4:1 or even of at least 5:1 and/or a manninotriose/manninotetraose weight ratio of 0.3:1 to 4:1, preferably of 0.8:1 to 1:1.

5. Non-therapeutic use, as prebiotic agent, of a water-soluble defructosylated pea extract according to either of Claims 1 and 2 or of a composition according to Claim 4.

6. Water-soluble defructosylated pea extract as defined in either of Claims 1 and 2 or a composition as defined in Claim 4, for the use thereof as prebiotic agent.

7. Carbonated beverage containing at least one sweetener and a water-soluble defructosylated pea extract according to either of Claims 1 and 2 and/or a composition according to Claim 4.

8. Foodstuff, such as a foodstuff with a long shelf life, a yoghurt or an infant milk, containing a water-soluble defructosylated pea extract according to either of Claims 1 and 2 and/or a composition according to Claim 4.
